Europäisches Patentamt

(19) European Patent Office

Office européen des brevets

(11) Veröffentlichungsnummer: **0 244 730**
B1

(12) # EUROPÄISCHE PATENTSCHRIFT

(45) Veröffentlichungstag der Patentschrift:
04.07.90

(21) Anmeldenummer: 87106094.3

(22) Anmeldetag: 27.04.87

(51) Int. Cl.⁵: **A61B 17/22**, A61B 6/00,
A61B 6/12

(54) **Einrichtung zum Zertrümmern von im Körper eines Lebewesens befindlichen Konkrementen.**

(30) Priorität: 09.05.86  DE 3615564

(43) Veröffentlichungstag der Anmeldung:
11.11.87 Patentblatt 87/46

(45) Bekanntmachung des Hinweises auf die Patenterteilung:
04.07.90 Patentblatt 90/27

(84) Benannte Vertragsstaaten:
DE FR GB NL

(56) Entgegenhaltungen:
EP-A- 0 081 051
DE-A- 2 722 252
DE-C- 496 749
GB-A- 2 002 987

(73) Patentinhaber: Siemens Aktiengesellschaft,
Wittelsbacherplatz 2, D-8000 München 2(DE)

(72) Erfinder: Grasser, Franz, Dipl.-Ing. (FH),
Neuwiesenstrasse 27, D-8557 Eggolsheim(DE)
Erfinder: Reitter, Josef, Elsterweg 3,
D-8521 Möhrendorf(DE)
Erfinder: Oppelt, Sylvester, Dipl.-Ing. (FH),
Greiffenbergstrasse 51, D-8600 Bamberg(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Einrichtung zum Zertrümmern von im Körper eines Lebewesens befindlichen Konkrementen mit einem Stoßwellengenerator und mit einer Röntgenuntersuchungsvorrichtung zur Ortung der Konkremente.

In der DE-OS 3 122 056 ist eine derartige Vorrichtung beschrieben, die beispielsweise zum Zertrümmern von Harnsteinen, Nierensteinen, Gallensteinen oder dergleichen dient. In einer Fokussierungskammer wird als Stoßwellengenerator eine Stoßwelle zum Beispiel durch Funkenentladung erzeugt, die auf das Konkrement konzentriert wird und es zerkleinert. Damit genau festgestellt werden kann, ob sich das Konkrement im Brennpunkt der Fokussierungskammer befindet, ist die Einrichtung mit einer Röntgenuntersuchungsvorrichtung zur besseren Ortung verbunden. Im Durchleuchtungsbetrieb werden die einzelnen Bilder eines Stereobildpaares in Bildspeicher einzeln oder über mehrere Bilder integriert im Fernsehtakt eingespeichert. Hierbei tritt aber das Problem auf, daß insbesondere bei der Behandlung von Nierensteinen diese während der Atmung bewegt werden, so daß eine genaue Lokalisierung der Konkremente nicht erfolgen kann.

In der EP-A 0 081 051 ist eine Auslösevorrichtung für Stoßwellen zu therapeutischen Zwecken, insbesondere zur Zerkleinerung von Nierensteinen beschrieben, die Abnehmer für periodische Körperfunktionen des Patienten, nämlich dessen Herz- und Atemtätigkeit, aufweist. Die Abnehmer sind über eine Auswerteschaltung mit einer Steuervorrichtung verbunden, die derart ausgebildet ist, daß sie Triggerimpulse zur Auslösung von Stoßwellen in Korrelation mit den periodischen Körperfunktionen zu solchen Zeitpunkten erzeugt, in denen keine Störungen der Körperfunktionen infolge der Stoßwellen auftreten können und eine erfolgreiche Steinzertrümmerung möglich ist. Im einzelnen erfolgt die Stoßwellenauslösung jeweils zu Zeitpunkten, in denen keine Herzrhythmusstörungen auftreten können und die Bewegungen des Konkrementes infolge der Atemtätigkeit des Lebewesens möglichst gering sind.

In der DE-C 496 749 ist eine Vorrichtung zur Anfertigung stereoskopischer Röntgenaufnahmen von sich periodisch bewegenden Organen eines Patienten beschrieben, die einen Abnehmer für die Atmung des Patienten aufweist, der die zur Anfertigung eines Stereo-Bildpaares erforderlichen Röntgenaufnahmen jeweils während der gleichen Atemphase des Patienten auslöst. Es ist so gewährleistet, daß die Lage des Organs bei der Anfertigung der ein Stereo-Bildpaar bildenden Röntgenaufnahmen jeweils die gleiche ist, so daß sich ein guter Stereoeindruck ergibt.

Die Erfindung geht von der Aufgabe aus, eine Vorrichtung der eingangs genannten Art zu schaffen, die es ermöglicht, eine sehr genaue Lokalisierung der Konkremente durchzuführen, so daß eine sichere, gewebeschonende Zerkleinerung der Konkremente erfolgt.

Die Aufgabe wird erfindungsgemäß dadurch gelöst, daß die Einrichtung wenigstens einen Abnehmer für periodische, eine Konkrementeverlagerung verursachende Körperfunktionen des Patienten aufweist, wobei der Abnehmer über eine Auswerteschaltung mit einer Steuervorrichtung verbunden ist, die derart ausgebildet ist, daß zur Ortung Röntgenbilder bei markanten Merkmalen der Körperfunktion erzeugt werden und daß nach erfolgter Ortung die Stoßwellen aufgrund von Triggerimpulsen ausgelöst werden, die den Zeitpunkten der Röntgenbilderzeugung zugeordnet sind. Durch die Betrachtung des Konkrementes beispielsweise bei einer bestimmten Atemphase, in der die zu untersuchende Region nahezu unbeweglich ist, lassen sich die Konkremente sehr genau lokalisieren, so daß die Einrichtung sich sehr genau einstellen läßt. Da weiterhin die Auslösung der Stoßwellen in der gleichen Phase erfolgt, wird somit eine sehr genaue Übereinstimmung des Lokalisationspunktes mit dem Brennpunkt des Stoßwellengenerators erreicht, so daß die gesamte aufgewandte Energie zur Zerkleinerung des Konkrementes genutzt werden kann.

Die Berücksichtigung der Atemphase kann erfolgen, wenn als Abnehmer ein Meßwertaufnehmer für die Atmung verwendet wird. Eine Bewegung innerhalb einer Phase wird sicher vermieden, wenn der Meßwertaufnehmer ein pneumatischer Gürtel ist. Die Herzfrequenz läßt sich zusätzlich berücksichtigen, wenn als weiterer Abnehmer eine EKG-Elektrode verwendet wird und die Triggerimpulse für die Röntgenbild- und Stoßwellenerzeugung über eine UND-Schaltung der Steuervorrichtung zugeführt werden.

Die Erfindung ist nachfolgend anhand eines in der Zeichnung dargestellten Ausführungsbeispieles näher erläutert.

Es zeigen:

Fig. 1 eine erfindungsgemäße Einrichtung, und
Fig. 2 und 3 Zeitverläufe zur Erläuterung der in Figur 1 dargestellten Einrichtung.

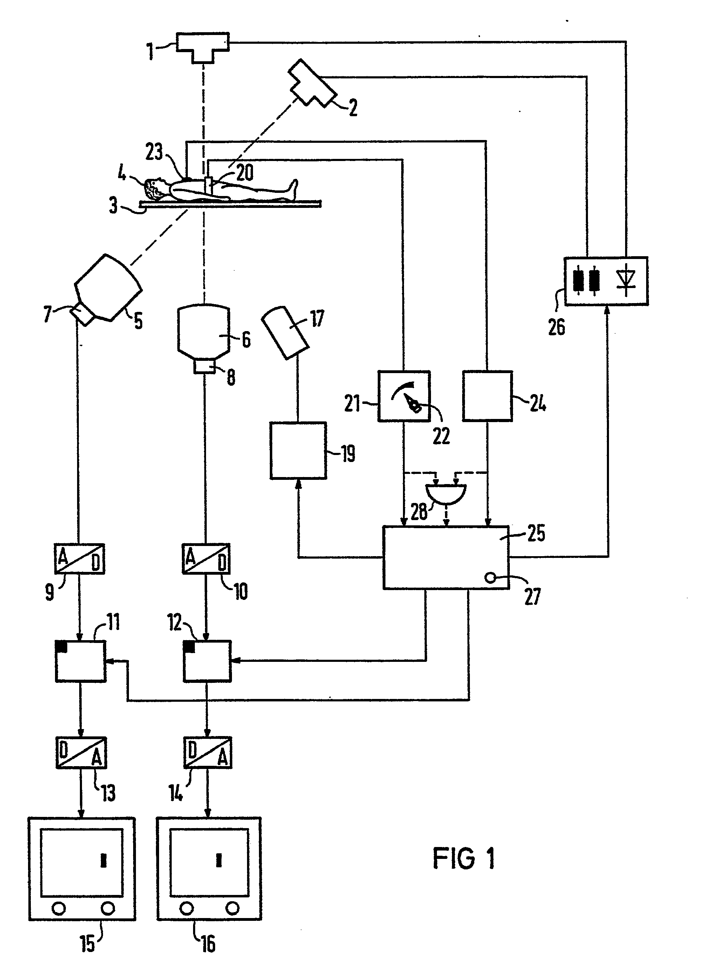

In der Figur 1 ist eine Einrichtung zum Zertrümmern von im Körper eines Lebewesens befindlichen Konkrementen mit einer Röntgenuntersuchungsvorrichtung dargestellt, die zwei Röntgenröhren 1 und 2 aufweist, die Röntgenstrahlenbündel erzeugen, die eine auf einer Patientenliege 3 befindliche, zu behandelnde Untersuchungsperson 4 durchdringen und auf die Eingangsleuchtschirme von Röntgenbildverstärker 5 und 6 fallen. Die Röntgenröhre 1 und der Röntgenbildverstärker 6 sind derart angeordnet, daß der Zentralstrahl des Röntgenstrahlenbündels der Röntgenröhre 1 senkrecht auf die Untersuchungsperson 4 fällt (a.p.-Projektion). Die Röntgenröhre 2 und der Röntgenbildverstärker 5 sind derart schräg angeordnet, daß der Zentralstrahl der Röntgenröhre 2 den Zentralstrahl der Röntgenröhre 1 in einem Zielgebiet innerhalb der Untersuchungsperson 4 unter einem Winkel von beispielsweise 45° schneidet. Dadurch erhält man Durchleuchtungsbilder aus zwei verschiedenen Projektionsrichtungen, so daß man die Untersuchungsperson 4 durch die Patientenliege 3 derart verschieben kann, daß sich in dem Zielgebiet die Konkremente befinden.

Die Ausgangssignale der an dem Röntgenbildverstärker 5 und 6 angekoppelten Fernsehkameras 7 und 8 werden über zwei Analog/Digital-Wandler (A/D-Wandler 9, 10) in zwei Bildspeicher 11 und 12 eingelesen. Die Ausgangssignale können über zwei Digital-Analog-Wandler (D/A-Wandler 13, 14) auf zwei Monitoren 15, 16 betrachtet werden. Befinden sich nun die Konkremente im Zielgebiet, so erscheinen sie in der Mitte der Bildschirme der Monitore 15 und 16.

Zur Zerkleinerung der in dem Zielgebiet befindlichen Konkremente ist ein Stoßwellengenerator 17 vorgesehen, der in Figur 1 schematisch dargestellt ist. Er erzeugt in bekannter Weise Stoßwellen, die zu einer Zertrümmerung der Konkremente führen, wenn sie beispielsweise im Brennpunkt des Stoßwellengenerators 17 liegen. Die Röntgenuntersuchungseinrichtung und der Stoßwellengenerator 17 sind derart fest miteinander verbunden, daß die Stoßwellen im Zielgebiet der Röntgenuntersuchungseinrichtung fokussiert sind. Der Stoßwellengenerator 17 ist an einer Ansteuerschaltung 19 angeschlossen.

An der Untersuchungsperson 4 ist ein Abnehmer 20 für die Atmung angebracht, der beispielsweise aus einem um den Bauch der Untersuchungsperson 4 geschlungenen pneumatischen Gürtel besteht. An dem Abnehmer 20 ist eine Schaltung 21 angeschlossen, die einen Einsteller 22 für einen Schwellwert aufweist. An der Untersuchungsperson 4 sind weiterhin EKG-Elektroden 23 angeordnet, die mit einer EKG-Schaltung 24 verbunden sind. Die Ausgangssignale der Schaltungen 21 und 24 werden einer Steuervorrichtung 25 zugeführt, die mit einem Hochspannungsgenerator 26 zur Steuerung der Röntgenröhren 1 und 2, mit den Bildspeichern 11 und 12 und der Ansteuerschaltung 19 für den Stoßwellengenerator 17 verbunden ist.

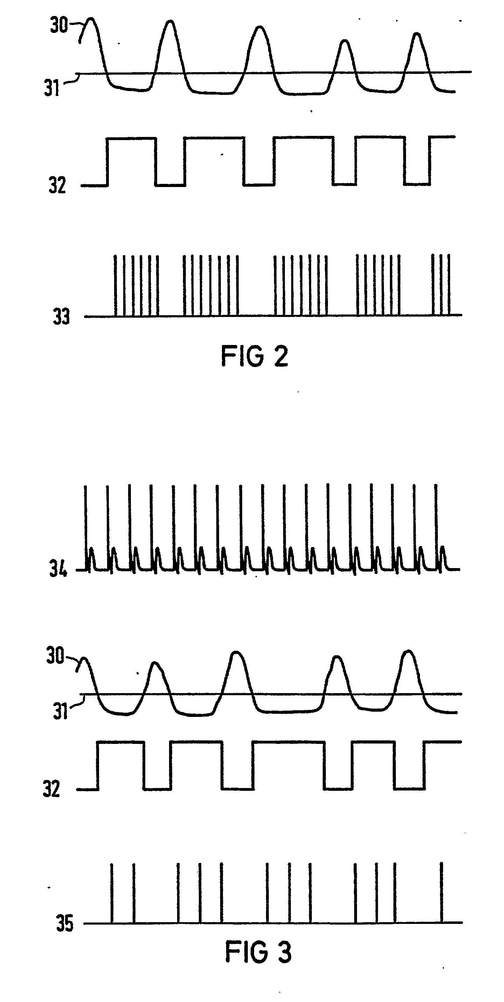

Anhand der in den Figuren 2 und 3 dargestellten Zeitverläufe wird die Funktionsweise der in Figur 1 dargestellten Einrichtung näher erläutert. Der Abnehmer 20 liefert ein der Atmung der Untersuchungsperson 4 entsprechendes Ausgangssignal 30. Eine durch den Einsteller 22 vorgewählte Schwelle 31 bewirkt beispielsweise durch einen Komparator ein Ausgangssignal 32 der Schaltung 21. Aufgrund beispielsweise der ansteigenden Vorderflanke der Rechteckimpulse oder auch zusätzlich zeitverzögert bewirkt die Steuervorrichtung 25 die Auslösung eines Röntgenimpulses durch den Hochspannungsgenerator 26 und die gleichzeitige Einspeicherung der Röntgenbilder in die Bildspeicher 11 und 12. Dadurch wird erreicht, daß immer in der gleichen Atemphase die Untersuchungsaufnahmen erstellt werden. Wird nun die Einrichtung durch einen Schalter 27 von Untersuchung auf Therapie umgeschaltet, so wird aus dem Ausgangssignal 32 der Schaltung 21 durch die Steuervorrichtung 25 ein Freigabesignal für die Ansteuerschaltung 19 des Stoßwellengenerator 17 erzeugt, der daraufhin innerhalb der Rechteckimpulse periodisch Stoßwellen erzeugt, die durch den Kurvenverlauf 33 als nadelförmige Impulse dargestellt sind. Es werden also während der Untersuchungsphase Röntgenbilder erstellt, die während einer bestimmten Atemphase anfallen, und während dieser gleichen Atemphase, in der nur eine geringe oder gar keine Bewegung beispielsweise der Niere erfolgt, werden mehrere Stoßwellen erzeugt, die die Konkremente gezielt treffen und somit nahezu vollständig zerkleinern.

Es kann aber auch gleichzeitig mit der Triggerung auf die Atemphase eine Triggerung aus dem EKG erfolgen, dessen Kurvenverlauf 34 in Figur 3 dargestellt ist. Hierzu werden aus dem EKG, beispielsweise aus der sogenannten R-Zacke, durch die EKG-Schaltung 24 Triggerimpulse erzeugt, die der Steuervorrichtung 25 zugeführt werden. Dieses Signal wird, wie gestrichelt dargestellt, zusammen mit dem Ausgangssignal 32 der Schaltung 21 in einem UND-Glied verkoppelt, so daß daraus ein Kombinationssignal entsteht, das zur Steuerung der Röntgenuntersuchungseinrichtung und des Stoßwellengenerators verwendet wird. Daraus folgt, daß nur während der durch das Ausgangssignal 32 bestimmten Atemphase und während des Auftretens der R-Zacke im EKG-Signal eine Aufnahme bzw. Stoßwelle erzeugt wird, wie dies aus dem in Figur 3 dargestellten Kurvenverlauf durch die Nadelimpulse 35 ersichtlich ist.

## Patentansprüche

1. Einrichtung zum Zertrümmern von im Körper eines Lebewesens befindlichen Konkrementen mit einem Stoßwellengenerator (17), mit einer Röntgenuntersuchungsvorrichtung (1 bis 16) zur Ortung des Konkrementes und mit wenigstens einem Abnehmer (20, 23) für periodische, eine Konkrementeverlagerung verursachende Körperfunktionen des Patienten (4), wobei der Abnehmer (20, 23) über eine Auswerteschaltung (21, 24) mit einer Steuervorrichtung (25) verbunden ist, die derart ausgebildet ist, daß zur Ortung Röntgenbilder bei markanten Merkmalen der Körperfunktion erzeugt werden und daß nach erfolgter Ortung die Stoßwellen aufgrund von Triggerimpulsen ausgelöst werden, die den Zeitpunkten der Röntgenbilderzeugung zugeordnet sind.

2. Einrichtung nach Anspruch 1, **dadurch gekennzeichnet**, daß als Abnehmer (20) ein Meßwertaufnehmer für die Atmung verwendet wird.

3. Einrichtung nach Anspruch 2, **dadurch gekennzeichnet**, daß der Meßwertaufnehmer (20) ein pneumatischer Gürtel ist.

4. Einrichtung nach Anspruch 2 oder 3, **dadurch gekennzeichnet**, daß als weiterer Abnehmer (23) eine EKG-Elektrode verwendet wird und daß die Triggerimpulse für die Röntgenbild- und Stoßwellenerzeugung über eine UND-Schaltung (28) der Steuervorrichtung (25) zugeführt werden.

## Claims

1. A device for crushing concretions located in the body of a living being having a shock-wave generator (17), an x-ray examining apparatus (1 to 16) for locating the concretion and with at least one receptor (20, 23) for periodic body functions which cause a concretion displacement in the patient (4), wherein the receptor (20, 23) is connected by way of an evaluating circuit (21, 24), to a control device (25), which is constructed such that for location, x-ray images are generated at pronounced features of the body function and in that after location is achieved the shock waves are released due to trigger pulses, which are allocated to the points in time of the x-ray image generation.

2. A device according to claim 1, characterised in that a measuring receptor for breathing is used as receptor (20).

3. A device according to claim 2, characterised in that the measuring receptor (20) is a pneumatic belt.

4. A device according to claim 2 or 3, characterised in that as further receptor (23) an EKG electrode is used and in that the trigger pulses for the x-ray image- and the shock wave generation are supplied by way of an AND gate (28) to the control device (25).

## Revendications

1. Dispositif pour détruire des concrétions situées dans le corps d'un être vivant, comportant un générateur d'ondes de choc (17), un dispositif d'examen radiographique (1 à 16) servant à localiser la concrétion et au moins un capteur (20, 23) servant à détecter des fonctions corporelles périodiques du patient (4), qui provoquent un décalage des concrétions, le capteur (20, 23) étant relié par l'intermédiaire d'un circuit d'évaluation (21, 24) à un dispositif de commande (25), agencé de telle sorte que, pour la localisation, des images radiographiques sont prises pour des caractéristiques marquantes de la fonction corporelle et, qu'une fois effectuée la localisation, les ondes de choc sont déclenchées au moyen d'impulsions de déclenchement qui sont associés aux instants de prise des images radiographiques.

2. Dispositif suivant la revendication 1, caractérisé par le fait qu'on utilise comme capteur un capteur de mesure de la respiration.

3. Dispositif suivant la revendication 2, caractérisé par le fait que le capteur de mesure (20) est une ceinture pneumatique.

4. Dispositif suivant la revendication 2 ou 3, caractérisé par le fait qu'on utilise comme autre capteur (23), une électrode d'enregistrement de l'électrocardiogramme et que les impulsions de déclenchement pour la prise des images radiographiques et la formation des ondes de choc sont envoyées par l'intermédiaire d'un circuit ET (28) au dispositif de commande (35).

FIG 1

FIG 2

FIG 3